(19) Europäisches Patentamt / European Patent Office / Office européen des brevets

(11) **EP 1 167 975 B1**

(12) **EUROPEAN PATENT SPECIFICATION**

(45) Date of publication and mention
of the grant of the patent:
**07.10.2009 Bulletin 2009/41**

(51) Int Cl.:
**G01N 33/574** (2006.01)    **C12Q 1/68** (2006.01)
**A61K 48/00** (2006.01)

(21) Application number: **00401778.6**

(22) Date of filing: **22.06.2000**

(54) **Compositions and methods for detecting, treating or predicting the response of breast tumor cells to endocrine therapy**

Zusammensetzungen und Methoden zum Nachweis, Behandeln oder Vorhersagen der Antwort von Brust Tumorzellen auf eine Endocrin-Therapie

Compositions et méthodes pour détecter, traiter ou prédire la réponse de cellules tumorales mammaires à une thérapie endocrine

(84) Designated Contracting States:
**AT BE CH CY DE DK ES FI FR GB GR IE IT LI LU MC NL PT SE**

(43) Date of publication of application:
**02.01.2002 Bulletin 2002/01**

(73) Proprietor: **UNIVERSITE PARIS DESCARTES**
**75270 Paris Cedex 06 (FR)**

(72) Inventors:
• **Vidaud, Michel**
  **94120 Fontenay Sous Bois (FR)**
• **Bieche, Ivan**
  **92150 Suresnes (FR)**

(74) Representative: **Becker, Philippe et al**
**Cabinet Becker & Associés**
**25, rue Louis Le Grand**
**75002 Paris (FR)**

(56) References cited:
**WO-A-97/40378**          **WO-A-97/49432**

• **DE CASTRO-BERNAS G ET AL: "Distribution profile of urinary human chorionic gonadotropin subunits: Assay development for lung and breast cancer." MEDICAL SCIENCE RESEARCH, vol. 26, no. 1, January 1998 (1998-01), pages 21-23, XP000974688 ISSN: 0269-8951**
• **MACFARLANE I A ET AL: "Serum glycoprotein hormone alpha subunit, hormone receptors and disease stage in patients with breast cancer." BRITISH JOURNAL OF CANCER, (1980 NOV) 42 (5) 645-50. , XP000974690**
• **RAVAIOLI A ET AL: "Prognosis and prediction of response in breast cancer: The current role of the main biological markers." CELL PROLIFERATION, vol. 31, no. 3-4, June 1998 (1998-06), pages 113-126, XP000974783 ISSN: 0960-7722**
• **KATZENELLENBOGEN BS, MONTANO MM, EKENA K, HERMAN ME, MCINERNEY EM: "Antiestrogens: mechanisms of action and resistance in breast cancer" BREAST CANCER RESEARCH AND TREATMENT, vol. 44, no. 1, May 1997 (1997-05), pages 23-38, XP000974689**
• **JOURNAL ARTICLE: 'Human Genome U95 Set' ANNOUNCEMENT AFFYMETRIX 01 April 2000, pages 1 - 2, XP002284454**

**Description**

The present invention relates to compositions and methods for predicting the responsiveness of tumor cells to therapy, as well as to methods and compositions for treating or detecting tumor cells *in vitro, ex vivo* or *in vivo*. The present invention relates more specifically to the identification of the gene coding for the alpha subunit of glycoprotein hormones (CGA) as a major endocrine-responsiveness gene or polypeptide and to any compositions and methods using CGA or any derivative or modulator thereof for treating, detecting or classifying tumors. The invention is more particularly suitable to predict or assess the endocrine treatment responsiveness of hormone-associated tumors, in particular breast or prostate tumors.

Breast cancer growth is regulated by estrogen, whose effect is mediated by binding to the estrogen receptor (ER). The presence of ER in breast tumors has thus been used to identify those patients who may respond to endocrine agents such as Tamoxifen. However, half the patients with ER-positive tumors fail to respond favourably to anti-oestrogen treatment (McGuire, 1980). Several mechanisms have been forwarded to explain the lack of response in ER-positive patients; one postulates a mutated, non-functional ER which is unable to mediate the inhibitory cellular actions of endocrine treatments (Katzenellenbogen et al., 1997). It has been postulated that co-assessment of ER and oestrogen-inducible genes or protein products (as markers of functional ER-mediated cellular growth mechanisms) might give better predictive results. In this regard, measurement of tumor progesterone receptor (PR) and PS2 contents have been shown to partly improve patient selection (Ribieras et al., 1998). However, expression of PR and PS2 correlate strongly with ER expression, meaning that the PS2 and PR markers provide little further information on hormone dependence relative to ER. Accordingly, there is a pressing need for more reliable markers of endocrine responsiveness. Such markers would further open novel strategies to detect or treat tumors, or to screen molecules that may impact on tumor development.

The present invention now discloses the identification of a novel marker of tumor cells responsiveness to endocrine therapy. More particularly, the present invention now discloses that CGA gene is an oestrogen receptor-responsive gene, whose expression correlates with tumor responsiveness to endocrine therapy. The present invention thus describes novel compositions and methods of predicting or assessing the responsiveness of a tumor cell to such endocrine treatments based on an analysis of the CGA status of said cell. The present invention also describes compositions and methods of predicting or assessing the endocrine treatment responsiveness of a subject having a hormone-mediated tumor, based on an analysis of the CGA status of said subject. As will be described in more details, the CGA status can be assessed in different ways, on tumor samples *ex vivo* or *in vitro,* as well as on samples of biological fluids. The invention also discloses novel methods of detecting tumor cells or tumor growth or formation, based on an assessment of CGA status in a subject. The invention also provides methods of treating tumor cells or reducing tumor growth in a subject based on the modulation of CGA gene expression in said cells, using either recombinant DNA techniques or synthetic molecules. In this regard, the invention further discloses methods for screening compounds that modulate CGA gene expression which represent new candidate compounds for reducing or inhibiting tumor cell formation or growth. The present invention also discloses reagents, such as nucleic acid probes, or antibodies, as well as kits for implementing the above methods.

As indicated above, this invention more particularly stems from the fortuitous and unexpected identification that the well-known CGA gene coding for the alpha subunit of glycoprotein hormones is a new ER-responsive gene in human hormone-associated cancer cells, in particular in human breast cancer cells. The alpha subunit is common to the four hormones belonging to a glycoprotein hormone family (i.e. hCG, LH, FSH and TSH). These hormones are composed of two non covalently bound subunits, designated $\alpha$ and $\beta$, only the $\beta$ subunit confers biological specificity. A first study was conducted by the inventors in order to determine if the emergence of "trophoblastic" CGB genes, that were previously shown to be associated with malignant breast transformation (Bièche et al., 1998), was accompanied by co-overexpression of the CGA gene and the production of ectopic human chorionic gonadotrophin (hCG) heterodimeric hormone in breast tumor cells. CGA and CGB gene expression was thus quantified in a large series of unilateral invasive primary breast tumor RNAs by means of a real-time quantitative RT-PCR assay. The inventors unexpectedly found marked differences in the total amount of CGA mRNA molecules, that were clearly independent of CGB expression status but fully associated with the ER-positive phenotype in the breast tumor series tested.

In particular, CGA gene overexpression was observed in half the ERa-positive tumors and in none of the ER$\alpha$-negative tumors. Immunohistochemical studies confirmed the CGA protein overexpression and showed that it was strictly limited to ER-positive tumor cells. To further examine the unexpected link between CGA gene status and ER positivity, we analyzed the expression of the oestrogen-responsive genes PR and PS2, as well as the oestrogen receptor $\beta$ (ER$\beta$). Our results indicate that CGA is a more reliable marker than PR and PS2 for ER functionality and, thus, for endocrine responsiveness. We infer that the subpopulation of CGA-positive, ER$\alpha$-positive tumors correspond to those responsive to hormone therapy. To support this, among the subgroup of ER$\alpha$-positive patients who received postoperative hormone therapy, more than 70% of ER$\alpha$-positive patients with CGA overexpression were relapse-free after endocrine treatment, while nearly 60% of patients with normal or no CGA expression relapsed (P=0.06). Taken together, these preliminary findings suggest that the CGA marker could provide new, independent information which, either alone or in conjunction with ERα status (or other markers), allows to determine the likelihood that a given tumor will respond to endocrine therapy.

To further validate CGA status as a new molecular predictor of endocrine responsiveness, CGA gene expression was next analyzed, as well as two well-known molecular markers (ERBB2 and CCND1 genes) in a second breast tumor RNA sample series (n=145) from a well-defined cohort of Tamoxifen-treated postmenopausal patients. By univariate analysis (log-rank test), we found that CGA-overexpressed patients had significantly longer relapse-free survival (RFS) (P=0.0098) while ERBB2-overexpressed patients had significantly shorter RFS (P=0.0085) compared to patients whose tumors normally expressed these two genes. We observed no link between RFS and CCND1 status. Only the good prognostic significance of CGA-overexpression status (and not the bad prognostic significance of ERBB2-overexpression status) persisted in Cox multivariate regression analysis (P=0.022 and P=0.18, respectively).

We also showed that combinations of CGA and ERBB2 status may provide an even more accurate predicting guide to hormone responsiveness. Indeed, analysis of both expression of CGA and ERBB2 genes allowed four separate prognostic groups to be identified (P=0.004). While CGA normal/ERBB2 overexpressed tumors showed the poorest prognosis, CGA overexpressed/ERBB2 normal tumors showed the best prognosis.

These results demonstrate the correlation between CGA status and endocrine treatment responsiveness of tumor cells. These results further show that CGA is a more reliable responsiveness marker than ER or PR or PS2 and provide the basis for improved methods of assessing a tumor cell responsiveness to endocrine therapy using CGA gene expression (or status) as a molecular marker. These results also allow the development of novel diagnostic and therapeutic approaches, based on the detection or modulation of CGA expression or activity.

In this respect, a first object of this invention resides more particularly in a method of predicting (or assessing or refining) endocrine treatment responsiveness of a subject with a breast tumor, comprising assessing the CGA status of said subject or tumor, *in vitro, ex vivo* or *in vivo.*

Another object of the present invention resides in a method of predicting (or assessing or refining) endocrine treatment responsiveness of a breast tumor cell, comprising assessing the CGA status of the tumor cell, *in vitro, ex vivo* or *in vivo.*

A further object of this invention comprises a method of selecting a patient with a breast tumor, for endocrine treatment, comprising assessing the CGA status of said patient or tumor.

More generally, the present invention relates to the use of a CGA gene product as a marker of endocrine responsiveness of a breast tumor or tumor cell.

Within the context of the present invention, a subject or patient includes any mammalian subject or patient, more preferably a human subject or patient. Also, the tumor is more generally any tumor at any stage of development, whether primary or metastatic. Endocrine treatment includes any therapeutic treatment of a tumor or subject in which an endocrine substance is used, either alone or in combination with any other treatment (chemical, radiation, etc.). Typical endocrine treatment comprises the administration of a hormone or hormone derivative or antagonist, such as the antiestrogens (Tamoxifen, Raloxifen, Droloxifen, Tonemifen, etc.), in particular in breast cancers.

The CGA status of a cell, tumor or subject can be assessed in various ways, more preferably by assessing (or determining or detecting or dosing) the presence or (relative) amount of a CGA gene expression product in a biological sample of the subject, in particular in a cell or tissue sample of said tumor or in a biological fluid of the subject, as will be described later.

In this regard, within the context of the present invention, CGA means the alpha subunit of glycoprotein hormones. This polypeptide and the corresponding coding nucleic acid have been described in the art (fiddes and Goodman, 1979 ; GenBank # V00518). In a more specific embodiment, a CGA polypeptide is a polypeptide having the amino acid sequence of SEQ ID NO: 2, or any variant or homolog thereof. Variants and homologs include any CGA polypeptide resulting from polymorphism, alterations or natural variations, such as splice variants, truncated forms, or other essentially naturally occurring polypeptides having amino acid substitutions or deletions. CGA polypeptide also includes variants of SEQ ID NO:2 which are recognized by antibodies specific for or raised by immunisation with a polypeptide of SEQ ID NO:2 or a fragment thereof. A CGA gene is any nucleic acid encoding a CGA polypeptide as described above. In a specific embodiment, a CGA gene is a genomic DNA comprising the nucleic acid sequence of SEQ ID NO:1 (GenBank # V00518), more particularly from residue 51 to residue 401 (e.g., the coding portion). In general terms, CGA gene also includes any cDNA, mRNA, artificial DNA as well as synthetic or semi-synthetic DNA encoding a CGA polypeptide.

As mentioned above, the invention comprises an assessment of the CGA status of a subject, breast tumor or tumor cell. The CGA status encompasses generally any assessment of the activity, regulation, or expression of CGA gene or of any other cellular component (such as genes, proteins, etc.) regulated or controlled by CGA. More particularly, assessing the CGA status of a cell, tumor or subject (or any biological sample derived therefrom) is accomplished by assessing (or determining or detecting or dosing) CGA gene expression in said cell, tumor or subject. In this regard, CGA gene expression may be assessed by assessment of the presence or (relative) amount of a CGA gene expression product in said sample, in particular any CGA transcription product (e.g., pre-RNAs, mRNAs) as well as any CGA translation product (e.g., polypeptides such as proteins, fragments thereof, peptides, etc.).

In this respect, in a first variant of the present invention, assessing CGA gene expression comprises detecting the presence or amount of CGA polypeptide in a biological sample of the subject.

In more specific embodiments, the assessment of CGA gene expression comprises:

- detecting the presence of CGA polypeptide in a cell or in a tissue sample of said tumor, or
- detecting the presence or amount of CGA polypeptide in a biological fluid from the subject. In this embodiment, the biological fluid may be blood, plasma or urine, for instance.

The presence or amount of a CGA polypeptide can be determined by any suitable technique known to the skilled artisan, including by immuno-assay (ELISA, EIA, RIA, etc.). This can be made using any affinity reagent specific for a CGA polypeptide, more preferably any antibody or fragment or derivative thereof. In a particular embodiment, the CGA polypeptide is detected with an anti-CGA antibody (or a fragment thereof), more preferably a monoclonal antibody. Such antibodies are commercially available (Biocode S.A., Immunotech, etc.). The antibody (or affinity reagent) may be labeled by any suitable method (radioactivity, fluorescence, enzymatic, chemical, etc.). Alternatively, CGA-antibody immune complexes may be revealed (and/or quantified) using a second reagent (e.g., antibody), labelled, that binds to the anti-CGA antibody, for instance.

CGA polypeptides are generally found within the cells. Accordingly, the CGA polypeptide detection or quantification usually comprises a treatment of the sample to render the polypeptide available to the affinity reagent. Treatment may comprise any conventional fixation technique, or any other conventional method used in immunohistology, for instance.

Typically, the sample is contacted with an anti-CGA antibody, and the immune complexes are detected or quantified. More preferably, where the sample is a cell or tissue sample, it is treated prior to contacting with the antibody, to alter the plasma membrane integrity and allow the antibodies to interact easily with CGA polypeptides.

This variant is particularly advantageous since CGA polypeptides are essentially absent from unresponsive tumoral tissues and from non-tumoral tissues, with the exception of some cells that produce the glycoprotein hormones (hCG, LH, FSH or/and TSH) such as pituitary cell types (gonadotropes and thyrotropes) as well as the placenta. Accordingly detection of the presence of a CGA polypeptide provides direct information as to the responsiveness potential of the tumor.

In a second variant of the present invention, assessing CGA gene expression comprises assessing CGA mRNA level in a cell or a tissue sample of said tumor, more preferably detecting or dosing CGA mRNAs in a tumor sample or cell or in a biological fluid (detection of circulating tumor cells). This can be made by any conventional method known to the skilled artisan, in particular by any method employing amplification (e.g. PCR), specific primers, specific probes, migration, etc. In a particular embodiment, CGA mRNA levels are assessed by quantitative RT-PCR. Alternatively, other nucleic acid detection methods can be employed, such as LCR (Ligase Chain Reaction), TMA (Transcription Mediated Amplification), PCE (an enzyme amplified immunoassay) and bDNA (branched DNA signal amplification) assays.

In a particular embodiment, mRNA level assessment comprises total RNA extraction from tumor cell or sample or biological fluid (blood, plasma, urine...) *in vitro* or *ex vivo,* cDNA synthesis, (PCR) amplification with specific CGA oligonucleotide primers, and analysis of PCR products.

In a typical embodiment, the CGA status is determined by:

- obtaining total RNAs of a tumor cell or sample or biological fluid (blood, plasma, urine...)
- cDNA synthesis by reverse transcription
- PCR (polymerase chain reaction) amplification of CGA cDNA in the presence of oligonucleotide primers specific for CGA gene.
- Detection (or quantitation) of PCR product, in particular by measuring the increase in fluorescence by using a fluorescent double-stranded DNA-binding dye such as the SYBR Green I (direct detection) or by next contact of a specific labelled nucleic acid probe to the CGA PCR products (indirect detection).

RT-PCR amplification of CGA mRNAs may be performed using any pair of CGA specific primers. Examples of such primers are SEQ ID NO: 3 and 4. It should be understood that other primers can be designed by the skilled artisan, such as any fragment of SEQ ID NO:1, for use in amplification step. The primers are preferably 15-25 bases long. Also, a specific CGA probe may be the nucleic acid of SEQ ID NO:1 or its complementary strand, or any fragment thereof, preferably any fragment of at least 15, more preferably at least 20-30 bases. A specific example of such a probe is SEQ ID NO:5. The probe may be labelled using any known technique such as radioactivity, fluorescence, enzymatic, chemical, etc. It should be understood that the present invention shall not be bound or limited by particular detection or labelling techniques, which can essentially be applied to the CGA gene using ordinary skills.

As mentioned above, the present invention allows the assessment, evaluation, or determination (or refining) of cancer responsiveness to endocrine therapy based on a CGA status evaluation. Generally, CGA overexpression is indicative of endocrine treatment responsiveness of said tumor or subject.

As indicated above, the CGA status can be assessed by a relative determination of CGA gene expression product contained in the biological sample. An increase in CGA expression products is generally indicative of a tumor (or tumor cell or subject) responsiveness to endocrine therapy. An increase may be determined by comparison to reference values or situations (in particular non-tumoral cells) or by the detection of a signal essentially absent from the control, non-cancerous tissue (in particular for CGA polypeptides).

In a particular embodiment, the presence of CGA mRNAs in the tumor cell or tissue sample is compared to an average value calculated from non-tumoral tissue sample (preferably of the same type), overexpression being indicative of endocrine treatment-responsiveness.

In a specific example, to determine the cut-off point for altered CGA gene expression at the RNA level in breast cancer tissue, the average normal CGA value (NCGA value) was determined for 15 normal breast tissue RNAs. This value consistantly fell between 0.4 and 6.6 (mean 1.83 $\pm$ standard deviation 1.78). Accordingly, values of 20 (mean + 10 SD) or more are considered to represent CGA gene overexpression in breast tumor RNA samples

More generally, an increase by at least two-fold, preferably at least five-fold, over control value is indicative of increased expression and predicts responsiveness.

The present invention may be used alone, or in combination with other markers such as ER$\alpha$, ERBB2, CCND1, PR or PS2. In this regard, the invention may be used to refine endocrine treatment responsiveness as assessed by a preliminary assay (i.e., be detecting ER$\alpha$ expression). In a more particular embodiment, this invention also relates to a method of predicting endocrine treatment responsiveness of a subject with a tumor, comprising assessing *in vitro* or *ex vivo* the ER$\alpha$ status and the CGA status of said tumor or subject.

An other object of this invention is a method of selecting a patient with a breast tumor, for endocrine treatment, comprising assessing the CGA status of said patient or tumor.

The present invention is more particularly suited for predicting, detecting or treating a hormone-associated breast tumor, which may benefit from a hormonal (or endocrine) treatment. Preferred examples include hormone-dependent tumors, i.e., tumors whose formation or development are known to be regulated by hormones, such as breast cancer, at any stage of development. In this regard, it is known that breast cancer growth is regulated by oestrogen, through binding to the oestrogen receptor. Current treatments include anti-oestrogen (endocrine) treatment, in order to antagonize the oestrogen-dependent tumor cell growth, such as for instance Tamoxifen treatment.

The present invention is used to predict responsiveness, or detect or treat hormone-dependent breast tumor or tumor cells.

A more specific object of this invention resides in a method of predicting Tamoxifen responsiveness of a subject with breast tumor, comprising assessing *in vitro* or *ex vivo* the CGA status of the subject or tumor.

**Materials And Methods**

1. Patients and Samples

We analyzed tissue from primary breast tumors excised from women treated at the Centre René Huguenin (St-Cloud, France). The samples were examined histologically for the presence of tumor cells. A tumor sample was considered suitable for this study if the proportion of tumor cells was more than 60%. Immediately following surgery the tumor samples were stored in liquid nitrogen until RNA extraction.
We analyzed two series of breast patients :

- The 131 patients of the first serie met the following criteria: primary unilateral non metastatic breast carcinoma on which complete clinical, histological and biological data were available; and no radiotherapy or chemotherapy before surgery.
- We also analyzed tissue from a additionnal well-defined cohort of 145 primary breast tumors. The patient of this second series met the additionnal following criteria : postmenopausal patients and treated after surgery only by endocrine therapy (Tamoxifen).

Specimens of adjacent normal breast tissue from nine of the breast cancer patients, and normal breast tissue from six women undergoing cosmetic breast surgery, were used as sources of normal RNA.

2. Real-time RT-PCR

(1) Theoretical basis

Quantitative values are obtained from the threshold cycle number at which the increase in the signal associated with exponential growth of PCR products begins to be detected using PE Biosystems analysis software, according to the manufacturer's manuals.

The precise amount of total RNA added to each reaction mix (based on optical density) and its quality (i.e. lack of extensive degradation) are both difficult to assess. We therefore also quantified transcripts of the *RPLP0* gene (also known as 36B4) encoding human acidic ribosomal phosphoprotein P0 as the endogenous RNA control, and each sample was normalized on the basis of its *RPLP0* content.

The relative target gene expression level was also normalized to a calibrator, or 1X sample, consisting of a normal breast tissue sample (quantitation of *CGA* and *RPLP0* genes) or of the breast tumor tissue sample which contained the smallest amount of target gene mRNA (quantitation of other target genes).

Final results, expressed as N-fold differences in target gene expression relative to the *RPLP0* gene and the calibrator, termed "N*target*", were determined as follows:

$$N target = 2^{(\Delta Ct_{sample} - \Delta Ct_{calibrator})}$$

where $\Delta Ct$ values of the sample and calibrator are determined by subtracting the average Ct value of the target gene from the average Ct value of the *RPLP0* gene.

*(2) Primers, probes and PCR consumables*

Primers and probes for the *RPLP0* and target genes were chosen with the assistance of the computer programs Oligo 4.0 (National Biosciences, Plymouth, MN) and Primer Express (Perkin-Elmer Applied Biosystems, Foster City, CA). We conducted BLASTN searches against dbEST and nr (the non redundant set of the GenBank, EMBL and DDBJ database sequences) to confirm the total gene specificity of the nucleotide sequences chosen for the primers and probes, and the absence of DNA polymorphisms. The nucleotide sequences of the oligonucleotide hybridization probes and primers are shown in Table 2. To avoid amplification of contaminating genomic DNA, one of the two primers was placed in a different exon. For example, the upper primer and the probe of *CGA* were placed in exon 3, whereas the lower primer was placed at the junction between exons 3 and 4.

*(3) RNA extraction*

Total RNA was extracted from breast specimens by using the acid-phenol guanidium method. The quality of the RNA samples was determined by electrophoresis through agarose gels and staining with ethidium bromide, and the 18S and 28S RNA bands were visualized under ultraviolet light.

*(4) cDNA Synthesis*

RNA was reverse transcribed in a final volume of 20 $\mu$l containing 1X RT buffer (500 mM each dNTP, 3 mM MgCl$_2$, 75 mM KCl, 50 mM Tris-HCl pH 8.3), 10 units of RNasin™ Ribonuclease inhibitor (Promega, Madison, WI), 10 mM dithiothreitol, 50 units of Superscript II RNase H⁻ reverse transcriptase (Gibco BRL, Gaithersburg, MD), 1.5 mM random hexamers (Pharmacia, Uppsala, Sweden) and 1 $\mu$g of total RNA. The samples were incubated at 20°C for 10 min and 42°C for 30 min, and reverse transcriptase was inactivated by heating at 99°C for 5 min and cooling at 5°C for 5 min.

*(5) PCR amplification*

All PCR reactions were performed using a ABI Prism 7700 Sequence Detection System (Perkin-Elmer Applied Biosystems). PCR was performed using either the TaqMan® PCR Core Reagents kit or the SYBR® Green PCR Core Reagents kit (Perkin-Elmer Applied Biosystems). The thermal cycling conditions comprised an initial denaturation step at 95°C for 10 min and 50 cycles at 95°C for 15 s and 65°C for 1 min. Experiments were performed with duplicates for each data point.

3. Immunohistochemical studies

Indirect immunoperoxidase staining of fixed tissues was performed using monoclonal antibody HT13 directed against the alpha subunit of human chorionic gonadotrophin (Bidart et al., 1988) and monoclonal antibody ID5 raised against human oestrogen receptor (Dako SA, Trappes, France).

The immunohistochemical procedure was performed on paraffin-embedded sections. A microwave antigen-retrieval technique was used in all cases. Sections were mounted on precoated slides (TESTA-coated slides) and allowed to dry at 50°C overnight. The sections were then dewaxed in xylene and hydrated through graded concentrations of alcohol. Endogeneous activity was blocked with 1% hydrogen peroxide for 15 min. Sections were then immersed in a thermoresistant plastic box containing 10 ml of pH 6.0 citrate buffer and processed in the microwave oven four times for 5 min each at 750 W. Sections were then allowed to cool at room temperature for 30 min before rinsing in TRIS-buffered saline. The blocking reagent was tipped off and the primary antibodies were left for one hour. A standard avidin-biotin-peroxidase complex was used to reveal the antibody-antigen reaction.

Localization and intensity of staining were assessed by two independent pathologists.

4. Statistical Analysis

Relapse-free survival (RFS) was determined as the interval between diagnosis and detection of the first relapse (local and/or regional recurrences, and/or metastases).

Clinical, histological and biological parameters were compared using the chi-square test. Differences between the two populations were judged significant at confidence levels greater than 95% (p<0.05). Survival distributions were estimated by the Kaplan-Meier method, and the significance of differences between survival rates was determined using the log-rank test.

**Examples**

1. CGA mRNA level in normal breast tissues

To determine the cut-off point for altered CGA gene expression at the RNA level in breast cancer tissue, the NCGA value, calculated as described in Materials and Methods, was determined for 15 normal breast tissue RNAs. As this value consistently fell between 0.4 and 6.6 (mean 1.83 $\pm$ standard deviation 1.78), values of 20 (mean + 10 SD) or more were considered to represent CGA gene overexpression in tumor RNA samples.

2. CGA mRNA level in the serie of 131 tumor breast tissues

Among the 131 breast tumor RNA samples tested, 44 (33.6%) showed CGA gene overexpression. Very large differences in the amount of CGA mRNA were observed (NCGA from 20.6 to 16500): 10 tumors had an expression level of 20 to 40 times, 11 tumors 40 to 100 times, 13 tumors 100 to 1000 times and 10 tumors more than 1000 times that of normal breast tissue RNA. The highest levels of CGA gene expression in breast tumors were close to those observed in placenta (data not shown). CGA gene expression was also investigated in nine patients from whom both primary breast tumors and matched normal breast tissue were available: four tumors showed clearly higher CGA expression in the tumor (NCGA = 2260.1, 70.4, 66.9 and 36.9, respectively) than in the normal tissue (NCGA = 0.9, 3.4, 2.2 and 1.3, respectively).

3. Correlation between CGA mRNA levels and clinical and pathological parameters.

We then sought links between quantitative CGA mRNA status and standard clinical, pathological and biological factors in breast cancer (Table 3).

Statistically significant links were found between CGA gene overexpression and Scarff-Bloom-Richardson (SBR) histopathological grade I+II (P=0.015), and positive progesterone (P=0.0009) and estrogen (P<10$^{-7}$) receptor status as determined by biochemical method (EIA). Importantly, we observed an absolute link between CGA gene overexpression and estrogen receptor status, all 44 patients with increased CGA mRNA levels being ER-positive.

Patients with tumors overexpressing CGA did not relapse more (or less) frequently (Table 3) and did not have significantly shorter (or longer) disease-free survival after surgery compared to patients whose tumors normally expressed CGA (log-rank test, not shown).

4. Localization of hCGα subunit in tumor cell cytoplasm

Of the 20 tumors studied by IHC, we detected specific immunoreactivity in the 10 tumors which overexpressed CGA mRNA and in none of the tumors which showed no CGA overexpression. We thus observed a perfect match between CGA mRNA overexpression and IHC positivity (Fig. 1) CGA immunoreactivity was exclusively found inside ER-positive tumor cells; infiltrating lymphocytes and normal glandular cells in the tumor were negative. Tumor cell staining was found exclusively in the cytoplasm, and the staining pattern consisted mainly of isolated positive cells (Fig. 1). The positive cells were chromogranin A-negative (data not shown).

5. Relationship between CGA mRNA levels and CGB mRNA levels.

Major differences in the amount of CGB mRNA molecules were observed (range from 0.3 to 192). However, the highest levels of CGB gene expression in breast tumors were >$10^3$-fold lower than those observed in placenta (data not shown). To study the relationship between CGA mRNA levels and CGB mRNA levels, we subdivided tumors into those with very low (NCGB from 0.3 to 2.9), low (3 to 5.9), intermediate (6 to 9.9) and high (10 to 192) CGB mRNA levels. No correlation was observed between CGA mRNA and CGB mRNA levels (Table 3). No links were observed either between CGB gene overexpression and progesterone and estrogen receptor status (data not shown).

6. Relationship between CGA mRNA levels and CCND1, MYC and ERBB2 expression status

The 131 tumors studied for CGA expression had previously been tested for mRNA expression of the CCND1, MYC and ERBB2 genes (Bièche et al., 1999a; 1999b and manuscript in preparation). We found a significant link between CGA overexpression and CCND1 overexpression (P=0.00003), but no link between CGA overexpression and altered mRNA expression of the other two genes (Table 3).

7. Relationship between CGA mRNA levels and PS2 and ERβ expression status

To study the link between CGA gene overexpression and ER positivity, we also quantified, by means of a real-time quantitative RT-PCR assay, the expression of the ERa-responsive gene PS2, as well as the estrogen receptor β (ERβ) gene. We also analyzed mRNA levels of the PR and ERα genes to confirm PR and ER protein status determined by a biochemical (EIA) method, to obtain quantitative PR and ERα values and to avoid a possible discrepancy due to tumor heterogeneity.

As regards the PS2, ERβ and PR markers, patients were subdivided into three equal groups of tumors with low (n=43), intermediate (n=44) and high (n=44) mRNA levels. A total correlation was observed between ERα gene status determined with biochemical and real-time RT-PCR. Indeed, all 41 ER-negative tumors in the biochemical (EIA) method had low ERα mRNA levels. Breast tumors were also subdivided into ERα-negative tumors (low ERα mRNA; n=43) and ERα-positive tumors (intermediate/high ERα mRNA; n=88).

The results (summarized in Tables 4 and 5) showed a stronger association between ERα gene status and CGA gene status (total link) than between ERα gene status and PS2, PR or ERβ gene status (subtotal links). Indeed, several ERa-negative tumors overexpressed PS2 and/or PR, while none of the ERα-negative (low ERα mRNA level) tumors overexpressed the CGA gene. Interestingly, we observed a negative correlation between ERα and ERβ mRNA expression levels (P=0.0013).

Finally, in the ERα-positive subgroup (n=88), we found a significant link between CGA overexpression and CCND1 overexpression (P=0.0024), but no link between CGA mRNA expression status and PS2, PR or ERβ mRNA expression levels (Table 6).

8. Correlation between CGA, ERBB2 and CCND1 mRNA levels and prognostic significance in a well-defined cohort of Tamoxifen-treated postmenopausal patients.

To further validate CGA status as a new molecular predictor of endocrine responsiveness, CGA gene, as well as two well-known molecular markers (ERBB2 and CCND1 genes) were analyzed in a second breast tumor RNA sample series (n = 145) from a well-defined cohort of Tamoxifen-treated postmenopausal patients.

By univariate analysis (log-rank test), we found that CGA-overexpressed patients had significantly longer relapse-free survival (RFS) (P=0.0098) while ERBB2-overexpressed patients had significantly shorter RFS (P=0.0085) compared to patients whose tumors normally expressed these two genes. We observed no link between RFS and CCND1 status.

Using a Cox proportional hazard model, we also assesed the prognostic value for RFS of parameters that were significant in univariate analysis, i.e., CGA and ERBB2 status. Only the good prognostic significance of CGA-overexpression status (and not the bad prognostic significance of ERBB2-overexpression status) persisted in Cox multivariate regression analysis (P=0.022).

We finaly tested if the combination of CGA and ERBB2 status may provide a more accurate predicting guide to hormone responsiveness than these individual factors used alone. By univariate analysis (log-rank test), we showed that analysis of both expression of CGA and ERBB2 gene allows four separate prognostic groups to be identified. The RFS curves are significantly different (p=0.004). CGA normal/ERBB2 overexpressed tumors showed the poorest prognosis, while CGA overexpressed/ERBB2 normal tumors showed the best prognosis. The two other groups (CGA normal/ERBB2 normal and CGA overexpressed/ERBB2 overexpressed tumors) showed similar intermediate outcome.

9. Discussion

In this report, we have developed a real-time quantitative RT-PCR assay to quantify CGA mRNA copy numbers in homogeneous total RNA solutions prepared from tumor samples. This recently developed approach to nucleic acid quantification in homogeneous solutions may become a reference, given its performance, accuracy, sensitivity, and high throughput capacity, and also the fact that it eliminates the need for tedious post-PCR processing (Gibson et al., 1996). Above all, this method is suited to the development of new target gene assays with a high level of inter-laboratory standardization, and yields statistical confidence values. A major advantage of this method is that the system has a large linear dynamic range (at least six orders of magnitude) suitable for analyzing the expression of genes such as CGA which show large differences in the amount of mRNA molecules (CGA gene expression ranged from 0.3 to 16500 times the level in normal breast tissues). Overexpression (> 10 SD above the mean for normal breast tissues) of the CGA gene was observed in 44 (33.6%) of the first serie of 131 breast tumor RNA samples. Immunohistochemical studies confirmed hCGα protein overexpression and showed that it was strictly limited to breast tumor cells. Our results are in keeping with a report of hCGa protein production by breast tumor cells, as shown by immunohistochemistry analysis (Walker, 1978).

We sought to determine whether the observed overexpression of hCGα protein in breast cancer has to aim to associate with the hCGβ subunit to produce the heterodimeric hormone hCG, which could mediate biological activities through the LH/hCG receptor which was recently shown to be expressed in breast cancer (Lojun et al., 1997; Meduri et al., 1997). Although we observed large differences in the amount of CGβ mRNA molecules (as high as 192 times the level in normal breast tissues), we found no link between CGA mRNA overexpression and CGB mRNA overexpression in our series of breast tumors (Table 3). We also observed no link between CGA overexpression and mRNA expression of the LHB, FSHB and TSHB genes, which code for the other three β subunits capable of associating with the hCGα subunit to produce glycoprotein hormones (data not shown).
The highest levels of CGA gene expression in our breast tumor series, which were close to those observed in placenta and were much higher than the amount of CGB mRNA in breast tumors (>$10^3$-fold lower that placental levels) suggest that different genetic (or epigenetic) mechanisms are responsible for CGA and CGB gene overexpression in breast tumors. Thus, involvement of the trophoblast-specific element (TSE) responsible for coordinated expression of the CGA and CGB genes in the placenta can be ruled out in breast tumors (Steger et al., 1993). Our results suggest that if the free hCGα subunit (and not the heterodimeric hormone hCG) is involved in breast tumorigenesis, it would act via a novel pathway not involving the LH/hCG receptor.

Little is known of the involvement of the hCGα subunit itself in carcinogenic processes. Rivera et al. (1989) showed that an antisense sequence to the CGA gene inhibited the growth and reversed the transformed phenotype of human lung tumor cells. One possibility, in breast tissue, is that the hCGα subunit controls PRL (prolactin) gene expression, as in myometrial, decidual and pituitary gland cells (Begeot et al., 1984, Blithe et al., 1991; Stewart et al., 1995). As PRL (prolactin) gene expression is altered in breast cancer (Goffin et al., 1999), we tested this hypothesis. Although we observed marked differences in the amount of PRL mRNA molecules, we found no link between CGA mRNA overexpression and PRL mRNA overexpression, ruling out a role of the CGA gene in PRL pathway dysregulation in breast tumors (data not shown).

The most important result of our study is that CGA gene overexpression is perfectly linked to estrogen receptor positivity in breast tumors. CGA gene overexpression was observed in half the ERα-positive tumors and in none of the ERα-negative tumors. We also found a weaker association between CGA gene overexpression and progesterone receptor positivity and SBR histophathological grade I+II, suggesting that CGA is a marker of tumors with low aggressiveness. No relationship between estrogen receptor positivity and CGB overexpression was observed in the same series. Our immunohistochemical data confirm that hCGα protein overexpression is strictly limited to estrogen receptor-positive tumor cells. This makes CGA status an attractive candidate as a molecular marker, and one that may prove far more reliable than estrogen receptor status alone, to predict endocrine responsiveness. Indeed, only half of all ER-positive patients respond favorably to antiestrogen treatment (McGuire, 1980) and we observed CGA gene overexpression in half the ERα-positive tumors in our breast tumor series. Moreover, we observed no link between CGA mRNA expression status and PS2 or PR mRNA expression levels in the ERα-positive subgroup, suggesting that CGA activation requires ERα but involves a mechanism different from that of PS2 and PR. Moreover, our results (summarized in Tables 4 and 5) suggest that the CGA gene may be a more reliable marker than PS2 and PR for ER functionality and, thus, for endocrine responsiveness. Indeed, several ERα-negative tumors overexpressed PS2 and/or PR, while none of the ERα-negative tumors overexpressed the CGA gene. Taken together, these findings suggest that the CGA marker provides new, independent information which, in conjunction with ER status, may help to determine the likelihood that a given tumor will respond to endocrine therapy. We infer that the subpopulation of CGA-positive, ER-positive tumors would correspond to those responsive to hormone therapy. As endocrine therapy was rarely used 20 years ago, when our samples were obtained, we were unable to study the relationship between CGA gene overexpression and the response to endocrine therapy in the first breast tumor series. However, among the 44 ER-positive patients who received postoperative hormone therapy, 71.4% (15/21) of those who had CGA overexpression are relapse-free, compared with 43.5% (10/23) of those who had normal CGA expression (P=0.06). In a second breast tumor RNA sample series from a well-defined cohort of Tamoxifen-treated postmenopausal patients, we confirm CGA status as a new attractive molecular predictor of endocrine responsiveness. Indeed, we found that CGA-overexpressed patients had significantly longer relapse-free survival compared to patients whose tumors normally expressed this gene, and this good prognostic significance of CGA-overexpression status persisted in Cox multivariate regression analysis. Finally, we also showed that combinations of CGA and ERBB2 status may provide a more accurate predicting guide to hormone responsiveness than these individual factors used alone. In particular, we observed that CGA normal/ERBB2 overexpressed tumors showed the poorest prognosis, while CGA overexpressed/ERBB2 normal tumors showed the best prognosis. Taken together, these findings identify CGA, in association or no with ERBB2, as an attractive new molecular marker for predicting hormone responsiveness in breast cancer.

In conclusion, we have identified CGA as a major new ERα-responsive gene that should greatly contribute to our understanding of the role of the estrogen receptor alpha in breast cancer. Our results also make the CGA gene an attractive alternative molecular marker to PR and PS2 as a (refined) predictor of endocrine responsiveness in breast cancer patients. The CGA gene encodes a well-characterized secreted protein for which specific antibodies are already commercially available, thus facilitating the application of this marker to the clinical management of breast cancer.

REFERENCES

Begeot et al. Science, 226: 566-568.
Bidart et al. J. Biol. Chem., 263: 10364-10369, 1988.
Bièche et al. Cancer Res., 59: 2759-2765, 1999a.
Bièche et al. Clin. Chem., 45: 1148-1156, 1999b.
Bièche, et al. Clin. Cancer Res., 4: 671-676, 1998.
Blithe et al. Endocrinology, 129: 2257-2259, 1991.
Fiddes JC and Goodman HM, Nature, 281:351-356, 1979.
Gibson et al. Genome Res., 6: 995-1001, 1996.
Goffin et al. Mol. Cell. Endocrinol, 151(1-2):79-87, 1999.
Katzenellenbogen et al. Breast Cancer Res. Treat., 44:23-38, 1997.
Lojun et al. Biol. Reprod., 57: 1202-10, 1997.
McGuire WL. Recent Prog. Horm. Res., 36:135-56, 1980.
Meduri et al. Cancer Res., 57: 857-864, 1997.
Ribieras et al. Biochim Biophys Acta, 1378: F61-77, 1998.
Rivera et al. J. Cell Biol., 108:2423-2434, 1989.
Steger et al. Mol. Endocrinol., 7: 1579-1588, 1993.
Stewart et al. Fertil. Steril., 64: 972-976, 1995.
Walker et al. , J. Clin. Pathol., 31: 245-249, 1978.

**Table 1:** Characteristics of the 131 patients and relation to disease-free survival

|  | | Disease-free survival | |
| --- | --- | --- | --- |
|  | Number of patients (%) | Number of events[1] | P value[2] |
| *Age* | | | NS |
| ≤50 | 39 (29.8) | 12 | |
| >50 | 92 (70.2) | 35 | |
| *Menopausal status* | | | NS |
| Premenopausal | 45 (34.3) | 16 | |
| Post-menopausal | 86 (65.7) | 31 | |
| *Histological grade[3]* | | | NS |
| I+II | 76 (62.3) | 30 | |
| III | 46 (37.7) | 16 | |
| *Lymph node status* | | | 0.026 |
| Node-negative | 49 (37.4) | 10 | |

(continued)

| | Number of patients (%) | Disease-free survival | |
| --- | --- | --- | --- |
| | | Number of events[1] | P value[2] |
| Node-positive | 82 (62.6) | 37 | |
| *ER status* | | | NS |
| + (≥ 10 fmlmg) | 90 (68.7) | 36 | |
| - (<10 fmlmg) | 41 (31.3) | 11 | |
| *PR status* | | | NS |
| + (≥ 10 fm/mg) | 78 (59.5) | 28 | |
| - (<10 fm/mg) | 53 (40.5) | 19 | |
| *Macroscopic tumor size[4]* | | | NS |
| ≤30mm | 90 (72.6) | 32 | |
| >30mm | 34 (27.4) | 13 | |

[1] : First relapses (local and/or regional recurrences, and/or metastases)

[2] : log-rank test

[3] : Scarff Bloom Richardson classification; Information available for 122 patients

[4] : Information available for 124 patients

**Table 2 :** Oligonucleotide primer and probe sequences used

| Genes | Oligonucleotide | Sequence | PCR product size (pb) |
| --- | --- | --- | --- |
| *RPLP0* | Upper primer | 5' - GGC GAC CTG GAA GTC CAA CT - 3' | 149 |
| | Lower primer | 5' - CCA TCA GCA CCA CAG CCT TC - 3' | |
| | Probe | 5' - ATC TGC TGC ATC TGC TTG GAG CCC A - 3' | |
| *CGA* | Upper primer[a] | 5' - TCC CAC TCC ACT AAG GTC CAA - 3' | 106 |
| | Lower primer[b] | 5' - CCC CAT TAC TGT GAC CCT GTT - 3' | |
| | Probe[c]= | 5' - CAC AGC AAG TGG ACT CTG AGG TGA CG - 3' | |
| *CGB* | Upper primer | 5' - GCT ACT GCC CCA CCA TGA CC - 3' | 94 |
| | Lower primer | 5' - ATG GAC TCG AAG CGC ACA TC - 3' | |
| | Probe | 5' - CCT GCC TCA GGT GGT GTG CAA CTA CC - 3' | |
| *ER*α* | Upper primer | 5' - CCA CCA ACC AGT GCA CCA TT - 3' | 108 |
| | Lower primer | 5' - GGT CTT TTC GTA TCC CAC CTT TC - 3' | |
| *PR** | Upper primer | 5' - CGC GCT CTA CCC TGC ACT C - 3' | 121 |
| | Lower primer | 5' - TGA ATC CGG CCT CAG GTA GTT - 3' | |
| *PS2** | Upper primer | 5' - CAT CGA CGT CCC TCC AGA AGA G - 3' | 105 |
| | Lower primer | 5' - CTC TGG GAC TAA TCA CCG TGC TG - 3' | |
| *ER*β* | Upper primer | 5' - AGA GTC CCT GGT GTG AAG CAA G - 3' | 143 |
| | Lower primer | 5' - GAC AGC GCA GAA GTG AGC ATC - 3' | |

* : The genes *ER*α, *PR, PS*2 and *RE*β were analyzed by using the fluorescent SYBR□ green methodology (without use of a TaqMan□ fluorogenic probe).

a: SEQ ID NO:3 b: SEQ ID NO:4 c: SEQ ID NO:5

**Table 3** : Relationship between mRNA *CGA* status (normal/overexpression) and the standard clinical, pathological, and biological factors

| | Total population (%) | Normal *CGA* mRNA<br>Number of patients (%) | Overexpressed *CGA* mRNA<br>Number of patients (%) | *P* value[1] |
|---|---|---|---|---|
| *Total* | 131 (100.0) | 87 (66.4) | 44 (33.6) | |
| *Age* | | | | NS |
| ≤50 | 39 (29.8) | 29 (33.3) | 10(20.7) | |
| >50 | 92 (70.2) | 58 (66.7) | 34 (77.3) | |
| *Menopausal status* | | | | NS |
| Premenopausal | 45 (34.3) | 33 (37.9) | 12 (27.3) | |
| Post-menopausal | 86 (65.7) | 54(62.1) | 32 (72.7) | |
| *Histological grade*[2] | | | | **0.015** |
| I+II | 76 (62.3) | 43 (54.4) | 33(76.7) | |
| III | 46 (37.7) | 36 (45.6) | 10 (23.3) | |
| *Lymph node status* | | | | NS |
| Node-negative | 49 (37.4) | 33 (37.9) | 16 (36.4) | |
| Node-positive | 82 (62.6) | 54 (62.1) | 28 (63.6) | |
| *ER status* | | | | $<10^{-7}$ |
| + (≥10 fm/mg) | 90 (68.7) | 46 (52.9) | 44(100.0) | |
| - (<10 fm/mg) | 41(31.3) | 41(47.1) | 0 | |
| *PR status* | | | | **0.0009** |
| + (≥10 fm/mg) | 78 (59.5) | 43 (49.4) | 35 (79.5) | |
| - (<10 fm/mg) | 53 (40.5) | 44 (50.6) | 9 (20.5) | |
| *Macroscopic tumor size* | | | | NS |
| ≤30mm | 90 (72.6) | 62 (73.8) | 28 (70.0) | |
| >30mm | 34 (27.4) | 22 (26.2) | 12 (30.0) | |
| *Relapses* | | | | NS |
| + | 47 (35.9) | 32 (36.8) | 15 (34.1) | |
| - | 84(64.1) | 55 (63.2) | 29 (65.9) | |
| *RNA CGB status* | | | | NS |
| NCGB (0.3 to 29) | 33 (25.2) | 25 (28.7) | 8 (18.2) | |
| NCGB (3.0 to 5.9) | 31 (23.7) | 20 (23.0) | 11 (25.0) | |
| NCGB (6.0 to 9.9) | 35 (26.7) | 23 (26.4) | 12 (27.3) | |
| NCGB (10.0 to 192) | 32 (24.4) | 19 (21.9) | 13 (29.5) | |
| *RNA CCND1 status*[4] | | | | **0.00003** |
| Overexpressed | 43 (32.8) | 18 (20.7) | 25 (56.8) | |
| Normal | 88 (67.2) | 69 (79.3) | 19 (43.2) | |
| *RNA MYC status*[5] | | | | N S |
| Overexpressed | 28 (21.4) | 17 (19.5) | 11 (25.0) | |
| Normal | 103 (78.6) | 70 (80.5) | 33 (75.0) | |
| *RNA ERBB2 status*[6] | | | | NS |
| Overexpressed | 22 (16.8) | 16 (18.3) | 6 (13.6) | |

(continued)

| | Total population (%) | Normal *CGA* mRNA | Overexpressed *CGA* mRNA | |
| --- | --- | --- | --- | --- |
| | | Number of patients (%) | Number of patients (%) | *P* value[1] |
| Normal | 109 (83.2) | 71 (81.7) | 38 (86.4) | |

[1] : Chi-square test

[2] : Scarff Bloom Richardson classification; Information available for 122 patients

[3] : Information available for 124 patients

[4] : Bièche et al., in preparation

[5] : Bièche et al. (1999a)

[6] : Bièche et al. (1999b)

**Table 4** : Relationship between mRNA *ER*α status and other molecular marker status

| | Total population | *ER*α **negative tumors** | *ER*α **moderately positive tumors** | *ER*α **highly positive tumors** | *P* value[1] |
| --- | --- | --- | --- | --- | --- |
| | | low *ER*α mRNA expressed tumors (N*ER*α: 1 to 40) [3] | intermediate *ER*α mRNA expressed tumors (N*ER*α: 41 to 300) | high *ER*α mRNA expressed tumors (N*ER*α: 301 to 50000) | |
| Total | 131 (100.0) | 43 (32.8)[2] | 44 (33.6) | 44 (33.6) | |
| *CGA* gene mRNA | | | | | <$10^{-7}$ |
| normal | 87 (66.4) | 43 (100.0) | 22 (50.0) | 22 (50.0) | |
| overexpressed | 44 (33.6) | 0 | 22 (50.0) | 22 (50.0) | |
| *PS2* gene mRNA | | | | | <$10^{-7}$ |
| low (N*PS2*: 1 to 70)3 | 43 (32.8) | 32 (74.4) | 5 (11.4) | 6 (13.6) | <$10^{-7}$ |
| intermediate (N*PS2*: 71 to 3000) | 44 (33.6) | 9 (20.9) | 19 (43.2) | 16 (36.4) | |
| high (N*PS2*: 3001 to 140000) | 44 (33.6) | 2 (4.7) | 20 (45.4) | 22 (50.0) | |
| *PR* gene mRNA | | | | | <$10^{-7}$ |
| low (N*PR*: 1 to 17) | 43 (32.8) | 32 (74.4) | 6 (13.6) | 5 (11.4) | |
| intermediate (N*PR*: 18 to 300) | 44 (33.6) | 10 (23.3) | 23 (52.3) | 12 (27.3) | |
| high (N*PR*: 301 to 12000) | 44 (33.6) | 1 (2.3) | 15 (34.1) | 27 (61.4) | |
| *RE*β gene mRNA | | | | | 0.0013 |
| low (N*ERB*: 1 to 16) | 43 (32.8) | 6 (14.0) | 21 (47.7) | 16 (36.4) | |
| intermediate (N*ERB*: 17 to 53) | 44 (33.6) | 13 (30.2) | 13 (29.6) | 18 (40.9) | |

(continued)

| | Total population | *ER*α **negative tumors** | *ER*α **moderately positive tumors** | *ER*α **highly positive tumors** | *P* value[1] |
|---|---|---|---|---|---|
| | | low *ER*α mRNA expressed tumors (N*ER*α: 1 to 40) [3] | intermediate *ER*α mRNA expressed tumors (N*ER*α: 41 to 300) | high *ER*α mRNA expressed tumors (N*ER*α: 301 to 50000) | |
| high (N*ERB*: 54 to 549) | 44 (33.6) | 24 (55.8) | 10 (22.7) | 10 (22.7) | |

[1] : Chi-square test (ERα-negative vs ERα-positive tumors; low vs intermediate/high gene mRNA expressed tumors)
[2] : Number of patients (%)
[3] : relative gene target expression value, normalized both to an endogenous RNA control (*RPLPO* gene) and to a calibrator (breast tumor tissue sample which contained the smallest amount of target gene mRNA

**Table 5** : Subdivision of the 131 tumors according to mRNA *CGA, ER*α, *PR* and *PS2* status.

| *CGA* status | *ER*α status | *PR* status | *PS2* status | Number of patients (%) |
|---|---|---|---|---|
| + | + | + | + | 35 (26.7) |
| + | + | + | - | 6 (4.6) |
| + | + | - | + | 2 (1.5) |
| + | + | - | - | 1 (0.8) |
| + | - | + | + | 0 |
| + | - | + | - | 0 |
| + | - | - | + | 0 |
| + | - | - | - | 0 |
| - | + | + | + | 33 (25.2) |
| - | + | + | - | 3 (2.3) |
| - | + | - | + | 7 (5.3) |
| - | + | - | - | 1 (0.8) |
| - | - | + | + | 8 (6.1) |
| - | - | + | - | 3 (2.3) |
| - | - | - | + | 3 (2.3) |
| - | - | - | - | 29 (22.1) |

- : low gene mRNA expression level
+ : intermediate or high gene mRNA expression level

**Table 6 :** Relationship between mRNA CGA status and other molecular marker status in the 88 ER positive tumors

| | Total population | CGA normal expressed - ER positive tumors | CGA overexpressed - ER positive tumors | *P* value[1] |
|---|---|---|---|---|
| Total | 88 (100.0) | 44 (50.0) | 44 (50.0) | |
| *PS2* gene mRNA | | | | NS |
| low | 11 (12.5)2 | 4 (9.1) | 7 (15.9) | |
| intermediate | 35 (39.8) | 19 (43.2) | 16 (36.4) | |

(continued)

| | Total population | CGA normal expressed - ER positive tumors | CGA overexpressed - ER positive tumors | P value[1] |
|---|---|---|---|---|
| high | 42 (47.7) | 21 (44.7) | 21 (47.7) | |
| *PR* gene mRNA | | | | NS |
| low | 11 (12.5) | 8 (18.2) | 3 (6.8) | |
| intermediate | 34 (38.6) | 12 (27.3) | 22 (50.0) | |
| high | 43 (48.9) | 24 (54.5) | 19 (43.2) | |
| *RE*β gene mRNA | | | | NS |
| low | 37 (42.1) | 15 (34.1) | 22 (50.0) | |
| intermediate | 31(35.2) | 17 (38.6) | 14(31.8) | |
| high | 20 (22.7) | 12 (27.3) | 8 (18.2) | |
| *CCND1* gene mRNA | | | | 0.0024 |
| normal | 52 (59.1) | 33 (75.0) | 19 (43.2) | |
| overexpressed | 36 (40.9) | 11 (25.0) | 25 (56.8) | |

[1] : Chi-square test
[2] : Number of patients (%)

**Table 7:** Relationship between appearance of relapses and mRNA CGA status (normal/overexpression) in 138 post-menopaused breast tumor patients who received postoperative hormone therapy

| | Total Population | No relapses | Relapses | |
|---|---|---|---|---|
| RNA *CGA* status | Number of patients | Number of patients (%) | Number of patients (%) | P value[1] |
| ***Total population*** | 138 | 93 (67.4) | 45 (32.6) | |
| CGA overexpression | 46 | 37 (80.4) | 9 (19.6) | **0.021** |
| CGA normal expression | 92 | 56 (60.9) | 36 (39.1) | |
| ***Subpopulation (age>70)*** | 63 | 42 (66.7) | 21 (33.3) | |
| CGA overexpression | 22 | 16 (72.7) | 6 (27.3) | **NS** |
| vCGA normal expression | 41 | 26 (63.4) | 15 (36.6) | |
| ***Subpopulation (age≤70)*** | 75 | 51 (68.0) | 24 (32.0) | |
| CGA overexpression | 24 | 21 (87.5) | 3 (12.5) | **0.013** |
| CGA normal expression | 51 | 30 (58.8) | 21 (41.2) | |

[1] : Chi-square test

**Claims**

1. A method of predicting endocrine treatment responsiveness of a subject with a breast cancer, comprising assessing *in vitro* or *ex vivo* the CGA gene encoding the alpha subunit of glycoprotein hormones or polypeptide expression of said subject or cancer.

**2.** A method of predicting endocrine treatment responsiveness of a breast cancer cell, comprising assessing *in vitro* or *ex vivo* the CGA gene or polypeptide expression of the breast cancer cell.

**3.** The method of claim 1 or 2, wherein assessing the CGA expression comprises assessing CGA gene expression in said subject or cancer.

**4.** The method of claim 3, wherein assessing CGA gene expression comprises detecting the presence or amount of CGA polypeptide in a biological sample of the subject.

**5.** The method of claim 4, wherein assessing CGA gene expression comprises detecting the presence of CGA polypeptide in a cell or in a tissue sample of said cancer.

**6.** The method of claim 4, wherein assessing CGA gene expression comprises detecting the presence or amount of CGA polypeptide in a biological fluid from the subject.

**7.** The method of any one of claims 4, 5 or 6, wherein the presence or amount of CGA polypeptide is determined by an immuno-assay, in particular using an anti-CGA antibody.

**8.** The method of claim 3, wherein assessing CGA gene expression comprises assessing CGA mRNA level in a cell or a tissue sample of said cancer.

**9.** The method of claim 3, wherein assessing CGA gene expression comprises assessing CGA mRNA level in biological fluids such as blood, plasma or urine.

**10.** The method of claim 8, comprising contacting the tumor cell or tissue sample with a labelled nucleic acid probe specific for the CGA mRNA under conditions allowing hybridization to occur, and dosing the labelled hybrids formed.

**11.** The method of claim 8 or 9, comprising a cDNA synthesis and a PCR amplification of the CGA mRNA with specific CGA oligonucleotide primers in the breast cancer cell, tissue sample or biological fluid (detection of circulating breast cancer cells) and a direct or indirect analysis of PCR products.

**12.** The method of claim 3, wherein the CGA expression is determined by:

- obtaining total RNAs of a breast cancer cell or sample or biological fluid
- cDNA synthesis by reverse transcription
- PCR (polymerase chain reaction) amplification of CGA cDNA in the presence of oligonucleotide primers specific for CGA gene,
- Detection (or quantitation) of PCR product by measuring the increase in fluorescence by using a fluorescent double-stranded DNA-binding dye or by subsequent contact of a specific labelled nucleic acid probe to the CGA PCR products.

**13.** The method of any one of claims 3 to 12, wherein CGA overexpression is indicative of endocrine treatment responsiveness of said cancer or subject.

**14.** The method of any one of claims 8 to 12, wherein the presence of CGA mRNAs in the breast cancer cell or tissue sample above an average value calculated from non-tumoral sample is indicative of endocrine treatment-responsiveness.

**15.** The method of claim 14, wherein a two-fold increase, preferably a five-fold increase, is indicative of endocrine responsiveness.

**16.** A method of predicting endocrine treatment responsiveness of a subject with a breast cancer, comprising assessing *in vitro* or *ex vivo* the ERα (estrogen receptor α) and the CGA gene or polypeptide expression of said cancer or subject.

**17.** A method of predicting the responsiveness to antiestrogens for a subject with breast cancer, comprising assessing *in vitro* or *ex vivo* the CGA gene or polypeptide expression of the subject or cancer.

**18.** The method of claim 17, wherein an increase in CGA expression over a reference value is indicative of a responsiveness to antiestrogens.

19. The method of claim 17 or 18, wherein the antiestrogen is selected from Tamoxifen, Raloxifen, Droloxifen and Tonemifen.

20. The use of a CGA gene product as a marker of endocrine treatment responsiveness of a breast cancer or a breast cancer tumor cell.

21. A method of selecting a patient with a breast cancer, for endocrine treatment, comprising assessing in vitro or ex vivo the CGA gene or polypeptide expression of said patient or cancer.

**Patentansprüche**

1. Verfahren zur Prognose des Ansprechens eines Subjekts mit Brustkrebs auf eine endokrine Behandlung, wobei das Verfahren eine *in vitro* oder *ex vivo* Beurteilung der CGA-Gen oder - Polypeptid Expression des genannten Subjekts oder Krebses umfasst, wobei das CGA-Gen die alpha-Untereinheit von Glykoproteinhormonen kodiert.

2. Verfahren zur Prognose des Ansprechens einer Brustkrebszelle auf eine endokrine Behandlung, wobei das Verfahren eine *in vitro* oder *ex vivo* Beurteilung der Expression des CGA-Gens oder -Polypeptids der Brustkrebszelle umfasst.

3. Verfahren nach Anspruch 1 oder 2, wobei die Beurteilung der CGA-Expression eine Beurteilung der CGA-Genexpression des genannten Subjekts oder Krebses umfasst.

4. Verfahren nach Anspruch 3, wobei die Beurteilung der CGA-Genexpression den Nachweis der Gegenwart oder Menge des CGA-Polypeptids in einer biologischen Probe des Subjekts umfasst.

5. Verfahren nach Anspruch 4, wobei die Beurteilung der CGA-Genexpression den Nachweis der Gegenwart des CGA-Polypeptids in einer Zelle oder Gewebeprobe des genannten Krebses umfasst.

6. Verfahren nach Anspruch 4, wobei die Beurteilung der CGA-Genexpression den Nachweis der Gegenwart oder Menge des CGA-Polypeptids in einer biologischen Flüssigkeit des Subjekts umfasst.

7. Verfahren nach einem der Ansprüche 4, 5 oder 6, wobei die Gegenwart oder Menge des CGA-Polypeptids durch einen Immun-Assay bestimmt wird, insbesondere durch die Verwendung eines Anti-CGA Antikörpers.

8. Verfahren nach Anspruch 3, wobei die Beurteilung der CGA-Genexpression eine Beurteilung des CGA-mRNA-Levels in einer Zelle oder einer Gewebeprobe des genannten Krebses umfasst.

9. Verfahren nach Anspruch 3, wobei die Beurteilung der CGA-Genexpression eine Beurteilung des CGA-mRNA-Levels in biologischen Flüssigkeiten wie Blut, Plasma oder Urin umfasst.

10. Verfahren nach Anspruch 8, wobei das Verfahren das in Kontakt bringen der Tumorzelle oder Gewebeprobe mit einer markierten Nukleinsäure-Sonde umfasst, wobei die Nukleinsäure-Sonde unter Bedingungen, die eine Hybridisierung ermöglichen, spezifisch für die CGA-mRNA ist, und wobei das Verfahren die Bemessung der entstandenen markierten Hybride umfasst.

11. Verfahren nach Anspruch 8 oder 9, wobei das Verfahren eine cDNA-Synthese und eine PCR-Amplifikation der CGA-mRNA mit spezifischen Oligonukleotid-Primern in der Brustkrebszelle, Gewebeprobe oder der biologischen Flüssigkeit (Nachweis von zirkulierenden Brustkrebszellen) und eine direkte oder indirekte Analyse von PCR-Produkten umfasst.

12. Verfahren nach Anspruch 3, wobei die CGA-Expression bestimmt wird durch:

    a) Erhalten von Gesamt-RNA einer Brustkrebszelle oder -probe oder biologischen Flüssigkeit;
    b) cDNA Synthese durch reverse Transkription;
    c) PCR (Polymerase-Kettenreaktion) Amplifikation von CGA-cDNA in Gegenwart von Oligonukleotid-Primern, die spezifisch für das CGA-Gen sind.

d) Nachweis (oder Quantifizierung) des PCR-Produkts durch Messung der Erhöhung der Fluoreszenz durch Verwendung eines Fluoreszenzfarbstoffs, der an doppelsträngige DNA bindet, oder durch nachträgliches in Kontakt bringen einer spezifischen, markierten Nukleinsäure-Sonde mit den CGA-PCR-Produkten.

13. Verfahren nach einem der Ansprüche 3 bis 12, wobei CGA-Überexpression auf ein Ansprechen des genannten Krebses oder Subjekts auf die endokrine Behandlung hindeutet.

14. Verfahren nach einem der Ansprüche 8 bis 12, wobei die Gegenwart der CGA-mRNAs in der Brustkrebszelle oder Gewebeprobe über einem Durchschnittswert, der aus nicht-Krebsproben berechnet wurde, auf ein Ansprechen auf die endokrine Behandlung hindeutet.

15. Verfahren nach Anspruch 14, wobei eine zweifache Erhöhung, vorzugsweise eine fünffache Erhöhung auf das Ansprechen auf die endokrine Behandlung hindeutet.

16. Verfahren zur Prognose des Ansprechens eines Subjekts mit Brustkrebs auf eine endokrine Behandlung, wobei das Verfahren eine *in vitro* oder *ex vivo* Beurteilung der Expression des ERα (Östrogenrezeptor α) und des CGA-Gens oder -Polypeptids des genannten Krebses oder Subjekts umfasst.

17. Verfahren zur Prognose des Ansprechens eines Subjekts mit Brustkrebs auf Anti-Östrogene, wobei das Verfahren eine *in vitro* oder *ex vivo* Beurteilung der Expression des CGA-Gens oder -Polypeptids des Subjekts oder Krebses umfasst.

18. Verfahren nach Anspruch 17, wobei ein Anstieg der CGA-Expression über einen Referenzwert auf ein Ansprechen auf Anti-Östrogene hindeutet.

19. Verfahren nach Anspruch 17 oder 18, wobei das Anti-Östrogen ausgewählt ist aus Tamoxifen, Raloxifen, Droloxifen und Toremifen.

20. Verwendung eines CGA-Genprodukts als Marker für das Ansprechen eines Brustkrebses oder einer Brustkrebstumorzelle auf eine endokrine Behandlung.

21. Verfahren zur Auswahl eines Patienten mit Brustkrebs für eine endokrine Behandlung, wobei die endokrine Behandlung eine *in vitro* oder *ex vivo* Beurteilung der Expression des CGA-Gens oder -Polypeptids des genannten Patienten oder Krebses umfasst.

**Revendications**

1. Méthode pour prédire la capacité de réponse à un traitement endocrine d'un sujet ayant un cancer du sein, comprenant l'évaluation in vitro ou ex vivo de l'expression du gène ou du polypeptide CGA dudit sujet codant la sous-unité alpha d'hormones glycoprotéiniques.

2. Méthode pour prédire la capacité de réponse à un traitement endocrine d'une cellule de cancer du sein, comprenant l'évaluation in vitro ou ex vivo de l'expression du gène ou du polypeptide CGA de ladite cellule de cancer du sein.

3. Méthode selon la revendication 1 ou 2, dans laquelle l'évaluation in vitro ou ex vivo de l'expression de CGA comprend l'évaluation de l'expression du gène CGA chez ledit sujet ou dans ladite cellule.

4. Méthode selon la revendication 3, dans laquelle l'évaluation de l'expression du gène CGA comprend la détection de la présence ou quantité de polypeptide CGA dans un échantillon biologique du sujet.

5. Méthode selon la revendication 4, dans laquelle l'évaluation de l'expression du gène CGA comprend la détection de la présence du polypeptide CGA dans un échantillon de cellule ou de tissu dudit cancer.

6. Méthode selon la revendication 4, dans laquelle l'évaluation de l'expression du gène CGA comprend la détection de la présence ou quantité de polypeptide CGA dans un fluide biologique du sujet.

**7.** Méthode selon l'une quelconque des revendications 4, 5 ou 6, dans laquelle la présence ou quantité de polypeptide CGA est déterminée par un immuno-essai, en particulier en utilisant un anticorps anti-CGA.

**8.** Méthode selon la revendication 3, dans laquelle l'évaluation de l'expression du gène CGA comprend l'évaluation du niveau d'ARNm CGA dans un échantillon de cellule ou de tissu dudit cancer.

**9.** Méthode selon la revendication 3, dans laquelle l'évaluation de l'expression du gène CGA comprend l'évaluation du niveau d'ARNm CGA dans des fluides biologiques tels que le sang, le plasma ou l'urine.

**10.** Méthode selon la revendication 8, comprenant la mise en contact de l'échantillon de tissu ou cellule de cancer avec une sonde d'acide nucléique marquée spécifique pour l'ARNm de CGA dans des conditions permettant la réalisation d'une hybridation, et le dosage des hybrides marqués formés.

**11.** Méthode selon la revendication 8 ou 9, comprenant une synthèse d'ADNc et une amplification PCR de l'ARNm de CGA avec des oligonucléotides amorces spécifiques de CGA dans la cellule de cancer du sein, l'échantillon de tissu ou le fluide biologique (détection de cellules cancéreuses circulantes) et une analyse directe ou indirecte des produits de PCR.

**12.** Méthode selon la revendication 3, dans laquelle l'expression du gène CGA est déterminée par :

- l'obtention des ARNs totaux de cellule ou échantillon de cancer du sein, ou de fluide biologique,
- synthèse d'ADNc par transcription inverse,
- amplification PCR (réaction de polymérase en chaîne) de l'ADNc de CGA en présence d'oligonucléotides amorces spécifiques du gène CGA,
- détection (ou quantification) du produit PCR par mesure de l'augmentation de fluorescence en utilisant un colorant fluorescent liant l'ADN double-brin ou par contact subséquent avec une sonde d'acide nucléique marquée spécifique pour l'ARNm des produits PCR de CGA.

**13.** Méthode selon l'une quelconque des revendications 3 à 12, dans laquelle une surexpression de CGA est indicative de la capacité à répondre au traitement endocrine dudit cancer ou sujet.

**14.** Méthode selon l'une quelconque des revendications 8 à 12, dans laquelle la présence de l'ARNm de CGA dans la cellule ou l'échantillon de tissu de cancer du sein au-delà d'une valeur moyenne calculée à partir d'échantillon non tumoral est indicative de la capacité à répondre au traitement endocrine.

**15.** Méthode selon la revendication 14, dans laquelle une augmentation d'un facteur 2, de préférence d'un facteur 5, est indicative de la capacité à répondre au traitement endocrine.

**16.** Méthode pour prédire la capacité de réponse à un traitement endocrine d'un sujet ayant un cancer du sein, comprenant l'évaluation in vitro ou ex vivo de l'expression de ERα (récepteur Estrogène α) et du gène ou du polypeptide CGA dudit sujet ou cancer.

**17.** Méthode pour prédire la capacité de réponse aux anti-estrogènes d'un sujet ayant un cancer du sein, comprenant l'évaluation in vitro ou ex vivo de l'expression du gène ou du polypeptide CGA dudit sujet ou cancer.

**18.** Méthode selon la revendication 17, dans laquelle une augmentation de l'expression de CGA par rapport à une valeur de référence est indicative de la capacité à répondre aux anti-estrogènes.

**19.** Méthode selon la revendication 17 ou 18, dans laquelle l'anti-estrogène est choisi parmi le Tamoxifène, Raloxifène, Droloxifène et le Tonemifène.

**20.** Utilisation d'un produit du gène CGA comme marqueur de la capacité à répondre au traitement endocrine d'un cancer du sein ou d'une cellule de cancer du sein.

**21.** Procédé pour sélectionner un patient ayant un cancer du sein, pour un traitement endocrine, comprenant l'évaluation in vitro ou ex vivo de l'expression du gène ou du polypeptide CGA dudit patient ou cancer.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Begeot et al.** *Science,* vol. 226, 566-568
- **Bidart et al.** *J. Biol. Chem.,* 1988, vol. 263, 10364-10369
- **Bièche et al.** *Cancer Res.,* 1999, vol. 59, 2759-2765
- **Bièche et al.** *Clin. Chem.,* 1999, vol. 45, 1148-1156
- **Bièche et al.** *Clin. Cancer Res.,* 1998, vol. 4, 671-676
- **Blithe et al.** *Endocrinology,* 1991, vol. 129, 2257-2259
- **Fiddes JC ; Goodman HM.** *Nature,* 1979, vol. 281, 351-356
- **Gibson et al.** *Genome Res.,* 1996, vol. 6, 995-1001
- **Goffin et al.** *Mol. Cell. Endocrinol,* 1999, vol. 151 (1-2), 79-87
- **Katzenellenbogen et al.** *Breast Cancer Res. Treat.,* 1997, vol. 44, 23-38
- **Lojun et al.** *Biol. Reprod.,* 1997, vol. 57, 1202-10
- **McGuire WL.** *Recent Prog. Horm. Res.,* 1980, vol. 36, 135-56
- **Meduri et al.** *Cancer Res.,* 1997, vol. 57, 857-864
- **Ribieras et al.** *Biochim Biophys Acta,* 1998, vol. 1378, F61-77
- **Rivera et al.** *J. Cell Biol.,* 1989, vol. 108, 2423-2434
- **Steger et al.** *Mol. Endocrinol.,* 1993, vol. 7, 1579-1588
- **Stewart et al.** *Fertil. Steril.,* 1995, vol. 64, 972-976
- **Walker et al.** *J. Clin. Pathol.,* 1978, vol. 31, 245-249